# EUROPEAN PATENT APPLICATION

(11) **EP 2 741 529 A2**
(43) Date of publication of application: **11.06.2014**
(21) Application number: 13190884.0
(22) Date of filing: 30.10.2013
(51) Int. Cl.: H04W 12/02, H04W 12/04

(54) **Data processing method, sensor device, and user terminal**

(30) Priority: 04.12.2012 KR 20120139348
(71) Applicant: Samsung Electronics Co., Ltd, Gyeonggi-do 443-742 (KR)
(72) Inventor: Kim, Jong Pal, Gyeonggi-do 446-712 (KR)
(74) Representative: Grünecker, Kinkeldey, Stockmair & Schwanhäusser

(57) **Abstract**

A data processing method performed by a sensor device, includes displaying identification information of the sensor device, and encrypting data measured by the sensor device based on the identification information. The method further includes transmitting the encrypted data to a user terminal.

## Description

### 1. Field

The following description relates to a data processing method, a sensor device, and a user terminal.

### 2. Description of Related Art

In association with a conventional method of encrypting sensor data to be transmitted, a security communication environment of personal health information may include a user terminal, a health management server, a security key issuing device, and a biosignal measuring device. The security key issuing device may generate an encryption key and a decryption key for the user teminal and the health management server, and transmit the encryption key and the decryption key to the user terminal and the health management server through local communication. The user terminal may obtain health information of a user, using the biosignal measuring device. The user terminal and the health management server may communicate through a network, and the user terminal may encrypt and transmit the health information of the user for security maintenance.

Use of a number of sensor devices operated while being attached to a body of a user has increased. A sensor device may measure a biosignal through being attached to the body of the user, and/or process the measured biosignal based on a series of processes.

The sensor device may transmit the measured bio signal to a user terminal. For example, an electrocardiogram (ECG) sensor device may transmit ECG information of the user to a mobile phone. As the sensor device transmits the biosignal to the user terminal, using a wireless communication, the sensor device may also transmit a biosignal to another user terminal. The biosignal of the user may be personal information, and the bio signal being transmitted to the other user terminal to be used may be unsuitable. Accordingly, there is a need for a method of processing sensor data between a sensor device used by a user and a user terminal.

### SUMMARY

In one general aspect, there is provided a data processing method performed by a sensor device, the method including displaying identification information of the sensor device, and encrypting data measured by the sensor device based on the identification information. The method further includes transmitting the encrypted data to a user terminal.

In another general aspect, there is provided a data processing method performed by a user terminal, the method including reading identification information of a sensor device that is displayed on the sensor device, and decoding data received from the sensor device based on the identification information.

In still another general aspect, there is provided a data processing method performed by a sensor device, the method including receiving identification information from a user terminal during a first mode, and converting the first mode to a second mode in response to the identification information being received, or a predetermined period of time elapsing. The method further includes encrypting data measured by the sensor device based on the identification information during a second mode, and transmitting the encrypted data to the user terminal.

In yet another general aspect, there is provided a data processing method performed by a user terminal, the method including transmitting identification information to a sensor device during a first mode, and converting the first mode to a second mode in response to the identification information being transmitted, or a predetermined period of time elapsing. The method further includes decoding data received from the sensor device based on the identification information during the second mode.

In another general aspect, there is provided a sensor device including an identification information displaying unit configured to display identification information of the sensor device, and a data measuring unit configured to measure data. The sensor device further includes a data encrypting unit configured to encrypt the data based on the identification information, and a data transmitting unit configured to transmit the encrypted data to a user terminal.

In still another general aspect, there is provided a user terminal including an identification information reading unit configured to read identification information of a sensor device that is displayed on the sensor device, and a data decoding unit configured to decode data received from the sensor device based on the identification information.

In yet another general aspect, there is provided a sensor device including an identification information receiving unit configured to receive identification information from a user terminal during a first mode, and a mode converting unit configured to convert the first mode to a second mode in response to the identification information being received, or a predetermined period of time elapsing. The sensor device further includes a data measuring unit configured to measure data during the second mode, and a data encrypting unit configured to encrypt the data based on the identification information. The sensor device further includes a data transmitting unit configured to transmit the encrypted data to the user terminal.

In another general aspect, there is provided a user terminal including an identification information transmitting unit configured to transmit identification information to a sensor device during a first mode, and a mode converting unit configured to convert the first mode to a second mode in response to the identification information being transmitted, or a predetermined period of time elapsing. The user terminal further includes a data decoding unit configured to decode data received from the sensor device based on the identification information during the second mode.

Other features and aspects will be apparent from the following detailed description, the drawings, and the claims.

### BRIEF DESCRIPTION OF THE DRAWINGS

FIG. 1 is a diagram illustrating an example of a sensor device transmitting sensor data to a user terminal.
FIG. 2 is a diagram illustrating an example of a sensor device.
FIG. 3 is a diagram illustrating an example of a user terminal.
FIG. 4 is a diagram illustrating another example of a sensor device.
FIG. 5 is a diagram illustrating another example of a user terminal.
FIGS. 6A and 6B are diagrams illustrating examples of sensor devices displaying identification information.
FIG. 7 is a flowchart illustrating an example of a data processing method performed by a sensor device.
FIG. 8 is a flowchart illustrating an example of a data processing method performed by a user terminal.
FIG. 9 is a flowchart illustrating another example of a data processing method performed by a sensor device.
FIG. 10 is a flowchart illustrating another example of a data processing method
performed by a user terminal.

### DETAILED DESCRIPTION

The following detailed description is provided to assist the reader in gaining a comprehensive understanding of the methods, apparatuses, and/or systems described herein. However, various changes, modifications, and equivalents of the systems, apparatuses and/or methods described herein will be apparent to one of ordinary skill in the art. Also, descriptions of functions and constructions that are well known to one of ordinary skill in the art may be omitted for increased clarity and conciseness.

Throughout the drawings and the detailed description, the same reference numerals refer to the same elements. The drawings may not be to scale, and the relative size, proportions, and depiction of elements in the drawings may be exaggerated for clarity, illustration, and convenience.

The features described herein may be embodied in different forms, and are not to be construed as being limited to the examples described herein. Rather, the examples described herein have been provided so that this disclosure will be thorough and complete, and will convey the full scope of the disclosure to one of ordinary skill in the art.

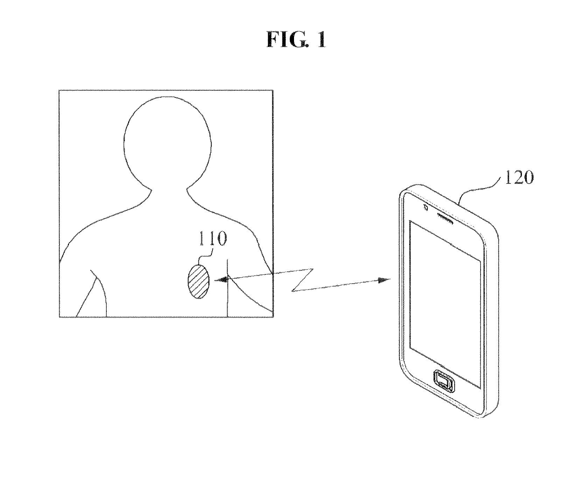

FIG. 1 illustrates an example of a sensor device 110 transmitting sensor data to a user terminal 120. The sensor device 110 measures the sensor data. For example, the sensor device 110 may measure bio information, such as an electrocardiogram (ECG), an electromyography (EMG), an electroencephalogram (EEG), and/or a body temperature, activity information, such as pausing, walking, running, lying down, and/or sitting down, and/or environmental information, such as a temperature, a humidity, a carbon dioxide (CO₂) density, and/or a ultraviolet (UV) ray density.

The sensor device 110 transmits the measured sensor data to the user terminal 120. For example, the sensor device 110 may be attached to a body of a user, measure a biosignal of the user, and transmit the biosignal to the user terminal 120 through wireless communication. The sensor device 110 may receive, from the user terminal 120, communication setting information, such as a gain, a bandwidth, a sampling rate, a communication channel, and/or other information known to one of ordinary skill in the art.

Also, the sensor device 110 performs a series of signal processing processes on the sensor data. For example, the sensor device 110 may perform, on the measured biosignal, the signal processing processes, such as amplifying, filtering, converting to a digital signal, and/or other processes known to one of ordinary skill in the art.

The user terminal 120 receives the sensor data from the sensor device 110. The user terminal 120 may be a mobile phone, a personal digital assistant (PDA), a tablet personal computer (PC), and/or other devices known to one of ordinary skill in the art; however, a type of the user terminal 120 is not limited to such devices. Also, the user terminal 120 may transmit, to the sensor device 110, the communication setting information to be used to perform the wireless communication with the sensor device 110.

The user terminal 120 analyzes the received sensor data, and outputs a result of the analysis. For example, the user terminal 120 may display the result of the analysis on a screen, and/or output the result of the analysis, using sound, vibration, and/or other methods known to one of ordinary skill in the art. Also, the user terminal 120 may store the sensor data, and/or transmit the sensor data to an external device.

In an example, the sensor device 110 may display identification information of the sensor device 110 to perform encrypted communication with the user terminal 120. For example, the sensor device 110 may convert the identification information to a visual code, and display the visual code on a screen. The sensor device 110 may encrypt the sensor data based on the identification information, and transmit the encrypted sensor data to the user terminal 120. The user terminal 120 may read the identification information displayed on the sensor device 110. The user terminal 120 may decode the encrypted sensor data received from the sensor device 110 based on the identification information.

In another example, the user terminal 120 may generate identification information in a first mode to perform encrypted communication with the sensor device 110, and transmit the generated identification information to the sensor device 110. The sensor device 110 in the first mode may receive the identification information from the user terminal 120. When the transmission of the identification information is completed, or a predetermined period of time elapses, the first mode may be converted to a second mode. The sensor device 110 in the second mode may encrypt sensor data based on the identification information received from the user terminal 120, and transmit the encrypted sensor data to the user terminal 120. The user terminal 120 in the second mode may decode the encrypted sensor data based on the identification information transmitted to the sensor device 110.

In either of the examples, the sensor device 110 performs the encrypted communication with the user terminal 120 based on the identification information. Accordingly, the sensor data measured by the sensor device 110 may be prevented from being transmitted to and be used in another user terminal.

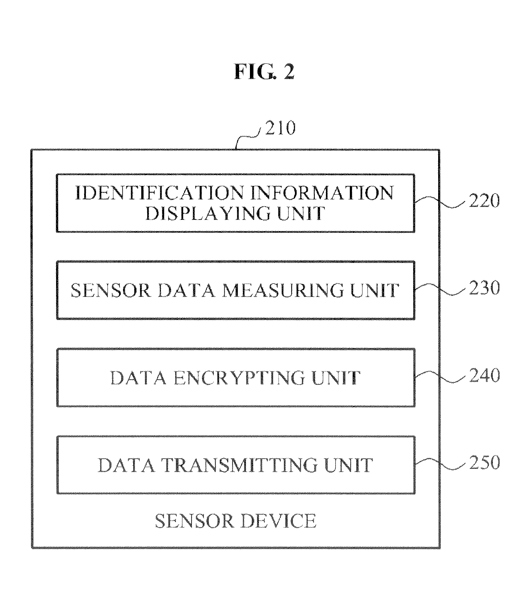

FIG. 2 illustrates an example of a sensor device 210. Referring to FIG. 2, the sensor device 210 includes an identification information displaying unit 220, a sensor data measuring unit 230, a data encrypting unit 240, and a data transmitting unit 250.

The identification information displaying unit 220 displays identification information of the sensor device 210 for a user terminal to process encrypted sensor data. For example, the identification information displaying unit 220 may display the identification information, using an electronic device, such as a liquid crystal display (LCD) device, or a non-electronic method, such as printing.

The identification information displaying unit 220 may display the identification information to be read by the user terminal during a predetermined period of time. Alternatively, the identification information displaying unit 220 may display the identification information until the sensor device 210 receives, from the user terminal, a signal indicating that reading, by the user terminal, of the identification information is completed.

The identification information of the sensor device 210 is assigned to the sensor device 210, to distinguish the sensor device 210 from other sensor devices. For example, the identification information may be a unique number arrangement or a unique character arrangement assigned to the sensor device 210. The identification information may be pre-stored in an identification information storing unit (not shown) of the sensor device 210. The identification information displaying unit 220 may display the identification information in an original form. Alternatively, the identification information displaying unit 220 may convert the identification information to another form, and display the other form. Accordingly, a form of the identification information displayed by the identification information displaying unit 220 may be dissimilar to the original form of the identification information.

The identification information displaying unit 220 may encode the identification information of the sensor device 210 to a visual code, and display the visual code as the identification information. For example, the visual code may include a barcode, a quick response (QR) code, a matrix code, a color code, and/or other codes known to one of ordinary skill in the art. However, a type of the visual code is not limited to those mentioned in the preceding.The identification information displaying unit 220 may display the identification information of the sensor device 210 by enumerating a symbol, such as a number, a letter, and/or other symbols known to one of ordinary skill in the art. The user terminal may read the identification information based on the symbol displayed on the sensor device 210. Alternatively, the user terminal may read the identification information based on a method of inputting, by a user, the symbol displayed on the sensor device 210 to the user terminal.

The sensor data measuring unit 230 measures sensor data. For example, the sensor data measuring unit 230 may measure bio information, such as an ECG and/or an EMG, activity information, such as walking and/or running, and/or environmental information, such as a temperature and/or a humidity. The sensor data measuring unit 230 may perform, on the measured sensor data, amplifying, filtering, and/or converting to a digital signal.

The data encrypting unit 240 encrypts the measured sensor data based on the identification information. For example, the data encrypting unit 240 may encrypt the measured sensor data based on a symmetric key method and/or other methods known to one of ordinary skill in the art, and based on the identification information to be used in the symmetric key method as an encryption key. In more detail, the data encrypting unit 240 may combine the measured sensor data and the identification information to convert the measured sensor data to a data arrangement corresponding to the encrypted sensor data, or convert the measured sensor data to a random form of data corresponding to the encrypted sensor data based on a predetermined function and/or the identification information.

The data transmitting unit 250 transmits the encrypted sensor data to the user terminal. The data transmitting unit 250 may transmit the encrypted sensor data to the user terminal based on communication setting information, such as a gain, a bandwidth, a sampling rate, a communication channel, and/or other information known to one of ordinary skill in the art, that is received from the user terminal.

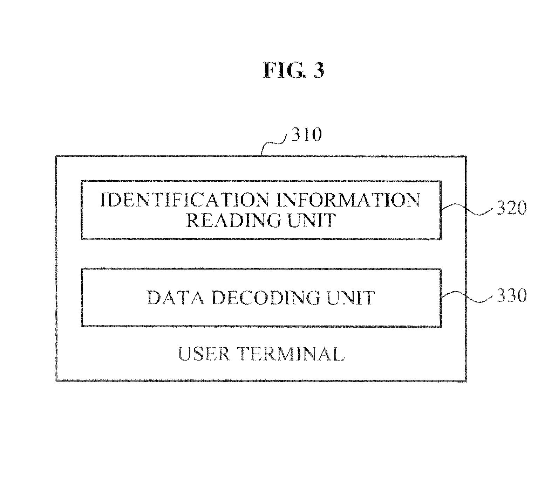

FIG. 3 illustrates an example of a user terminal 310. Referring to FIG. 3, the user terminal 310 includes an identification information reading unit 320 and a data decoding unit 330.

The identification information reading unit 320 may read identification information of a sensor device that is displayed on the sensor device. The identification information reading unit 320 may include, e.g., a camera, a scanner, a reader, and/or other devices known to one of ordinary skill in the art. For example, the identification information reading unit 320 may capture an image of the identification information displayed on the sensor device, using the camera. Subsequently, the identification information reading unit 320 may extract the identification information from the captured image.

Alternatively or additionally, the identification information reading unit 320 may read the identification information of the sensor device based on user input information. For example, a user may input, directly to the user terminal 310, the identification information displayed on the sensor device, and the identification information reading unit 320 may read the identification information of the sensor device based on the user input information inputted by the user. In this example, if "1234" is displayed on the sensor device, the user may input "1234" directly to the user terminal 310, and the identification information reading unit 320 may recognize identification information corresponding to "1234" inputted by the user.

The data decoding unit 330 decodes encrypted sensor data received from the sensor device based on the identification information. For example, the data decoding unit 330 may set the identification information to be a decoding key to be used to decode the encrypted sensor data.

The data decoding unit 330 may decode the encrypted sensor data based on data decoding information corresponding to the identification information. The data decoding information may include information of a method of decoding the encrypted sensor data. The data decoding information may be pre-stored in the user terminal 310, and/or be received from an external server. For example, the user terminal 310 may transmit the identification information to the external server, and receive, from the external server, the data decoding information corresponding to the identification information. Subsequently, the data decoding unit 330 may decode the encrypted sensor data based on the received data decoding information.

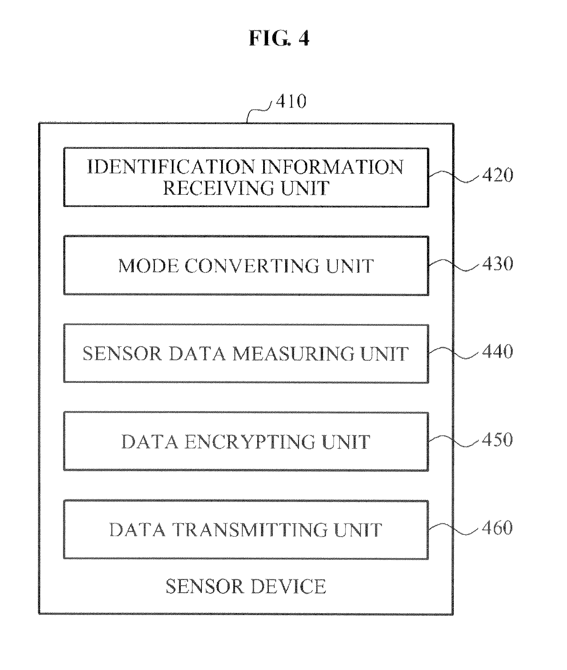

FIG. 4 illustrates another example of a sensor device 410. Referring to FIG. 4, the sensor device 410 includes an identification information receiving unit 420, a mode converting unit 430, a sensor data measuring unit 440, a data encrypting unit 450, and a data transmitting unit 460.

The identification information receiving unit 420 receives identification information from a user terminal during a first mode. The identification information is to be used by the sensor device 410 and the user terminal to perform encrypted communication with each other. The identification information may be generated by the user terminal based on a random method, a predetermined method, or user input information. For example, the identification information may be generated by the user terminal based on a number, a letter, or a combination of numbers and/or letters, that is inputted by a user to the user terminal. Alternatively, the identification information may be generated by the user terminal based on predetermined information delivered to the user terminal from an external server or an external device, and/or pre-stored in the user terminal. The identification information receiving unit 420 may store the received identification information in an identification information storing unit (not shown) of the sensor device 410.

When the reception of the identification information is completed, or a predetermined period of time elapses, the mode converting unit 430 converts the first mode to a second mode. The sensor device 410 may operate in the first mode during the predetermined period of time that begins in response to the sensor device 410 being initialized, or to a distribution of power to the sensor device 410 being authorized. Also, the sensor device 410 may operate in the first mode until the identification information is received from the user terminal.

The sensor data measuring unit 440 measures sensor data during the second mode. For example, the sensor data measuring unit 440 may measure bio information, such as an ECG and/or an EMG, activity information, such as walking and/or running, and/or environmental information such as a temperature and/or a humidity. The sensor data measuring unit 440 may perform, on the measured sensor data, atnplifying, filtering, or converting to a digital signal.

The data encrypting unit 450 encrypts the measured sensor data during the second mode based on the identification information. For example, the data encrypting unit 450 may set the identification information to be an encryption key to be used to encrypt the sensor data. The data encrypting unit 450 may combine the measured sensor data and the identification information to convert the measured sensor data to a data arrangement corresponding to the encrypted sensor data, or convert the measured sensor data to a random form of data corresponding to the encrypted sensor data based on a predetermined function and/or the identification information.

The data transmitting unit 460 transmits the encrypted sensor data to the user terminal.

The data transmitting unit 460 may transmit the encrypted sensor data to the user terminal based on communication setting information, such as a gain, a bandwidth, a sampling rate, a communication channel, and/or other information known to one of ordinary skill in the art, that is received from the user terminal.

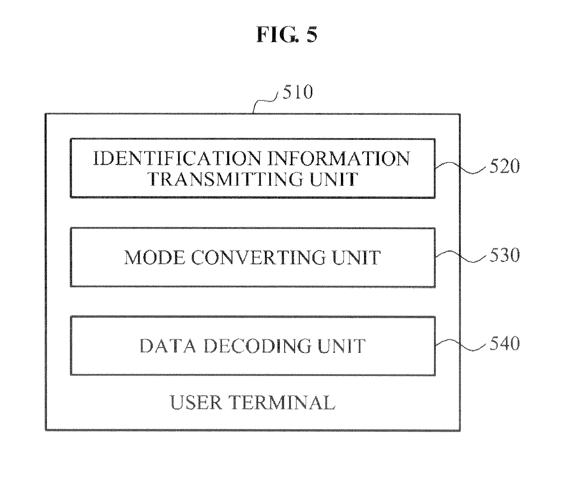

FIG. 5 illustrates another example of a user terminal 510. Referring to FIG. 5, the user terminal 510 includes an identification information transmitting unit 520, a mode converting unit 530, and a data decoding unit 540.

The identification information transmitting unit 520 transmits identification information to a sensor device during a first mode. The user terminal 510 may generate the identification information based on a random method or a predetermined method. Alternatively, the user terminal 510 may generate the identification information based on user input information or predetermined information delivered to the user terminal 510 from an external server and/or an external device.

The user terminal 510 may operates in the first mode during a predetermined period of time that begins in response to initialization of the sensor device being detected. Alternatively, the user terminal 510 may operate in the first mode until the user terminal 510 receives an acknowledgement (ACK) signal from the sensor device that indicates that the sensor device receives the identification information from the user terminal 510.

The mode converting unit 530 converts the first mode to a second mode when the transmission of the identification information is completed, or the predetermined period of time elapses. For example, the mode converting unit 530 may convert the first mode to the second mode automatically when the mode converting unit 530 receives the ACK signal from the sensor device that indicates that the sensor device receives the identification information from the user terminal 510, or when the predetermined period of time elapses.

The data decoding unit 540 decodes encrypted sensor data received from the sensor device during the second mode based on the identification information. For example, the data decoding unit 540 may set the identification information to be a decoding key to be used to decode the encrypted sensor data.

The data decoding unit 540 may decode the encrypted sensor data based on data decoding information corresponding to identification information. The data decoding information may include information of a method of decoding the encrypted sensor data. The data decoding information may be pre-stored and/or received from an external server. For example, the user terminal 510 may transmit the identification information to the external server, and receive, from the external server, the data decoding information corresponding to the identification information. Subsequently, the data decoding unit 540 may decode the encrypted sensor data based on the received data decoding information.

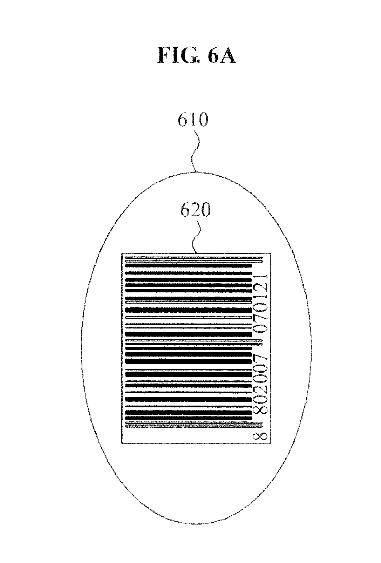

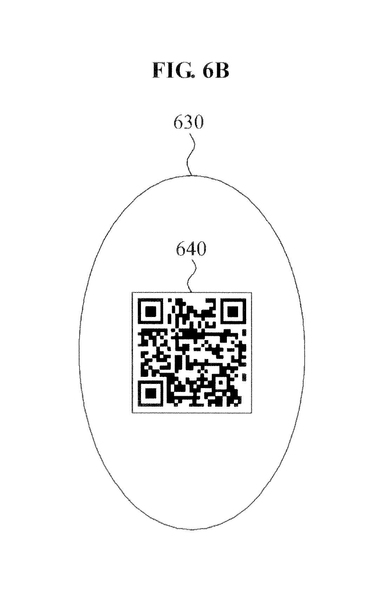

FIGS. 6A and 6B illustrate examples of sensor devices 610 and 630 displaying identification information. In FIG. 6A, the sensor device 610 displays the identification information of the sensor device 610 in a form of a barcode 620. The identification information in the form of the barcode 620 may be uniquely assigned to the sensor device 610. The user terminal may recognize the barcode 620 displayed on the sensor device 610, and read the identification information of the sensor device 610 from the barcode 620. Alternatively, a user may input a number arrangement included in the barcode 620 to the user terminal directly, and the user terminal may read the identification information of the sensor device 610 based on user input information, namely, the input number arrangement. The user terminal may decode encrypted sensor data received from the sensor device 610 based on the read identification information.

In FIG. 6B, the sensor device 630 displays the identification information of the sensor device 630 in a form of a QR code 640. The identification information in the form of the QR code 640 may be uniquely assigned to the sensor device 630. The user terminal may recognize the QR code 640 displayed on the sensor device 630, using a camera and/or other devices known to one of ordinary skill in the art, and read the identification information of the sensor device 630 from the QR code 640. The user terminal may decode the encrypted sensor data received from the sensor device 630 based on the read identification information.

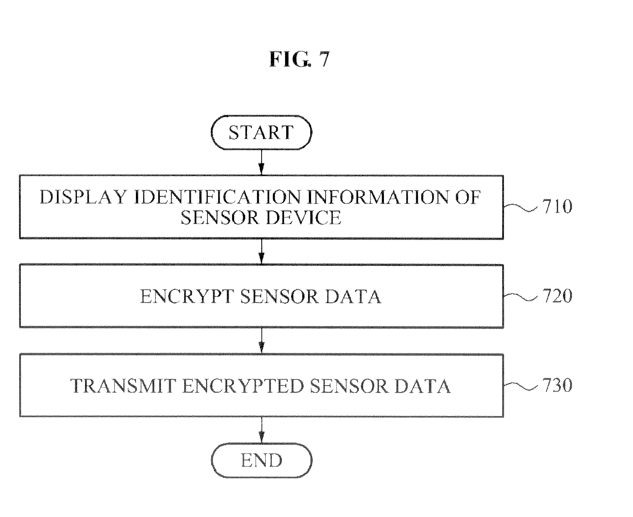

FIG. 7 illustrates an example of a data processing method performed by a sensor device. In operation 710, the sensor device displays identification information of the sensor device for a user terminal to process encrypted sensor data. For example, the sensor device may display the identification information, using an electronic device, such as an LCD device, or a non-electronic method, such as printing.

The sensor device may display the identification information during a predetermined period of time for the identification information to be read by the user terminal. Alternatively, the sensor device may display the identification information until the sensor device receives a signal from the user terminal that indicates that reading, by the user terminal, of the identification information is completed.

The sensor device may encode the identification information to a visual code, such as a barcode, a QR code, and/or other codes known to one of ordinary skill in the art, and display the visual code as the identification information. The sensor device may enumerate symbols, such as a number, a letter, and/or other symbols known to one of ordinary skill in the art, to display the identification information.

In operation 720, the sensor device encrypts sensor data based on the identification information. For example, the sensor device may encrypt the sensor data based on a symmetric key method and/or other methods known to one of ordinary skill in the art, and based on the identification information to be used in the symmetric key method as an encryption key. The sensor device may combine the sensor data and the identification information to convert the sensor data to a data arrangement corresponding to the encrypted sensor data, or convert the sensor data to a random form of data corresponding to the encrypted sensor data based on a predetermined function and/or the identification information. In operation 730, the sensor device transmits the encrypted sensor data to the user terminal. The sensor device may transmit the encrypted sensor data to the user terminal based on communication setting information, such as a gain, a bandwidth, a sampling rate, a communication channel, and/or other information known to one of ordinary skill in the art, that is received from the user terminal.

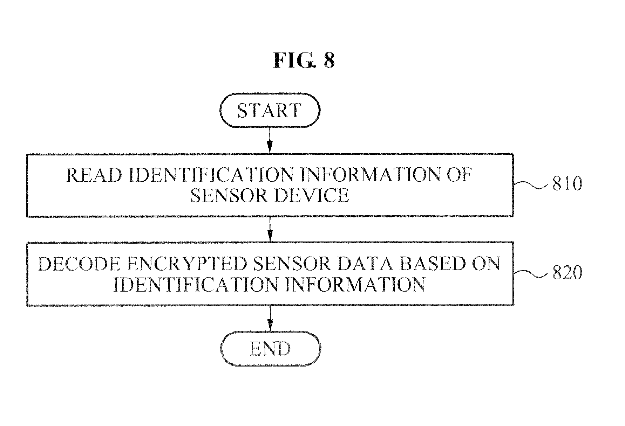

FIG. 8 illustrates an example of a data processing method performed by a user terminal. In operation 810, the user terminal reads identification information of a sensor device that is displayed on the sensor device. For example, the user terminal may capture an image of the identification information displayed on the sensor device, using a camera, a scanner, and/or other devices known to one of ordinary skill in the art, and read (e.g., extract) the identification information from the captured image. Alternatively, the sensor device may read the identification information based on user input information. For example, a user may input, directly to the user terminal, the identification information displayed on the sensor device as the user input information.

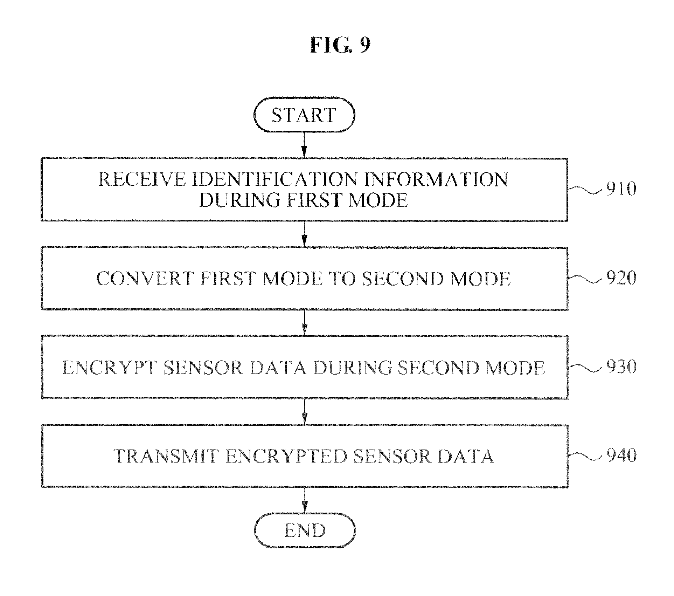

In operation 820, the user terminal decodes encrypted sensor data received from the sensor device based on the identification information. For example, the user terminal may set the identification information to be a decoding key to be used to decode the encrypted sensor data. The user terminal may decode the encrypted sensor data based on data decoding information corresponding to the identification information. The data decoding information may be pre-stored in the user terminal, and/or received from an external server. FIG. 9 illustrates another example of a data processing method performed by a sensor device. In operation 910, the sensor device receives identification information from a user terminal during a first mode. The identification information may be generated by the user terminal based on a predetermined method or user input information. For example, the identification information may be generated by the user terminal based on a number, a letter, or a combination of numbers and/or letter that are inputted by a user to the user terminal. Alternatively, the identification information may be generated by the user terminal based on predetermined information delivered to the user terminal from an external server and/or an external device, and/or pre-stored in the user terminal.

In operation 920, the sensor device converts the first mode to a second mode when the reception of the identification information is completed, or a predetermined period of time elapses. The sensor device may operate in the first mode for the predetermined period of time that begins in response to the sensor device being initialized. Alternatively, the sensor device may operate in the first mode until the identification information is received from the user terminal.

In operation 930, the sensor device encrypts sensor data during the second mode based on the identification information. For example, the sensor device may set the identification information to be an encryption key to be used to encrypt the sensor data. The sensor device may combine the sensor data and the identification information to convert the sensor data to a data arrangement corresponding to the encrypted sensor data, or convert the sensor data to a random form of data corresponding to the encrypted sensor data based on a predetermined function and/or the identification information.

In operation 940, the sensor device transmits the encrypted sensor data to the user terminal. The sensor data may transmit the sensor data to the user terminal based on communication setting information, such as a gain, a bandwidth, a sampling rate, a communication channel, and/or other information known to one of ordinary skill in the art, that is received from the user terminal.

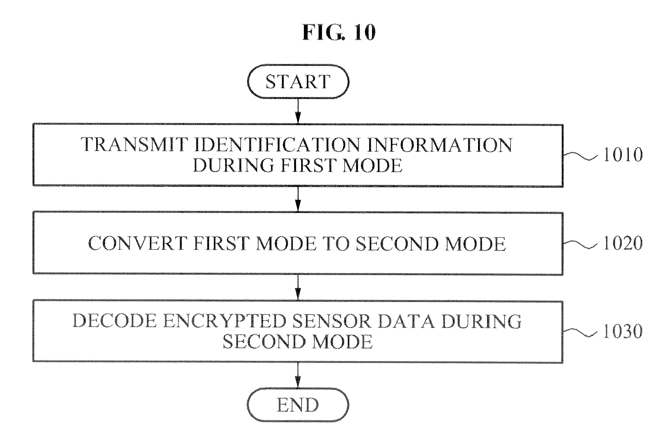

FIG. 10 includes another example of a data processing method performed by a user terminal. In operation 1010, the user terminal transmits identification information to a sensor device during a first mode. The user terminal may generate the identification information based on a predetermined method, or user input information. Alternatively, the identification information may be generated based on predetermined information delivered from an external server and/or an external device to the user terminal, and/or be pre-stored in the user terminal.

The user terminal may operate in the first mode for a predetermined period of time that begins in response to initialization of the sensor device being detected. Alternatively, the user terminal may operate in the first mode until the user terminal receives an acknowledgement (ACK) signal from the sensor device that indicates that the sensor device receives the identification information from the user terminal.

In operation 1020, the user terminal converts the first mode to a second mode when the transmission of the identification information is completed, or the predetermined period of time elapses. For example, the user terminal may convert the first mode to the second mode automatically if the user terminal receives the ACK signal from the sensor device that indicates that the sensor device receives the identification information from the user terminal, or if the predetermined period of time elapses.

In operation 1030, the user terminal decodes encrypted sensor data received from the sensor device during the second mode based on the identification information. For example, the user terminal may set the identification information to be a decoding key to be used to decode the encrypted sensor data. Alternatively, the user terminal may decode the encrypted sensor data based on data decoding information corresponding to the identification information. The data decoding information may be pre-stored in the user terminal, and/or be received from the external server.

The various units and methods described above may be implemented using one or more hardware components, one or more software components, or a combination of one or more hardware components and one or more software components.

A hardware component may be, for example, a physical device that physically performs one or more operations, but is not limited thereto. Examples of hardware components include microphones, amplifiers, low-pass filters, high-pass filters, band-pass filters, analog-to-digital converters, digital-to-analog converters, and processing devices.

A software component may be implemented, for example, by a processing device controlled by software or instructions to perform one or more operations, but is not limited thereto. A computer, controller, or other control device may cause the processing device to run the software or execute the instnictions. One software component may be implemented by one processing device, or two or more software components may be implemented by one processing device, or one software component may be implemented by two or more processing devices, or two or more software components may be implemented by two or more processing devices.

A processing device may be implemented using one or more general-purpose or special-purpose computers, such as, for example, a processor, a controller and an arithmetic logic unit, a digital signal processor, a microcomputer, a field-programmable array, a programmable logic unit, a microprocessor, or any other device capable of running software or executing instmctions. The processing device may run an operating system (OS), and may run one or more software applications that operate under the OS. The processing device may access, store, manipulate, process, and create data when running the software or executing the instructions. For simplicity, the singular term "processing device" may be used in the description, but one of ordinary skill in the art will appreciate that a processing device may include multiple processing elements and multiple types of processing elements. For example, a processing device may include one or more processors, or one or more processors and one or more controllers. In addition, different processing configurations are possible, such as parallel processors or multi-core processors.

A processing device configured to implement a software component to perform an operation A may include a processor programmed to run software or execute instructions to control the processor to perform operation A. In addition, a processing device configured to implement a software component to perform an operation A, an operation B, and an operation C may include various configurations, such as, for example, a processor configured to implement a software component to perform operations A, B, and C; a first processor configured to implement a software component to perform operation A, and a second processor configured to implement a software component to perform operations B and C; a first processor configured to implement a software component to perform operations A and B, and a second processor configured to implement a software component to perform operation C; a first processor configured to implement a software component to perform operation A, a second processor configured to implement a software component to perform operation B, and a third processor configured to implement a software component to perform operation C; a first processor configured to implement a software component to perform operations A, B, and C, and a second processor configured to implement a software component to perform operations A, B, and C, or any other configuration of one or more processors each implementing one or more of operations A, B, and C. Although these examples refer to three operations A, B, C, the number of operations that may implemented is not limited to three, but may be any number of operations required to achieve a desired result or perform a desired task.

Software or instructions that control a processing device to implement a software component may include a computer program, a piece of code, an instruction, or some combination thereof, that independently or collectively instructs or configures the processing device to perform one or more desired operations. The software or instructions may include machine code that may be directly executed by the processing device, such as machine code produced by a compiler, and/or higher-level code that may be executed by the processing device using an interpreter. The software or instructions and any associated data, data files, and data structures may be embodied permanently or temporarily in any type of machine, component, physical or virtual equipment, computer storage medium or device, or a propagated signal wave capable of providing instructions or data to or being interpreted by the processing device. The software or instructions and any associated data, data files, and data structures also may be distributed over network-coupled computer systems so that the software or instructions and any associated data, data files, and data structures are stored and executed in a distributed fashion.

For example, the software or instructions and any associated data, data files, and data structures may be recorded, stored, or fixed in one or more non-transitory computer-readable storage media. A non-transitory computer-readable storage medium may be any data storage device that is capable of storing the software or instructions and any associated data, data files, and data structures so that they can be read by a computer system or processing device. Examples of a non-transitory computer-readable storage medium include read-only memory (ROM), random-access memory (RAM), flash memory, CD-ROMs, CD-Rs, CD+Rs, CD-RWs, CD+RWs, DVD-ROMs, DVD-Rs, DVD+Rs, DVD-RWs, DVD+RWs, DVD-RAMs, BD-ROMs, BD-Rs, BD-R LTHs, BD-REs, magnetic tapes, floppy disks, magnetooptical data storage devices, optical data storage devices, hard disks, solid-state disks, or any other non-transitory computer-readable storage medium known to one of ordinary skill in the art.

Functional programs, codes, and code segments that implement the examples disclosed herein can be easily constructed by a programmer skilled in the art to which the examples pertain based on the drawings and their corresponding descriptions as provided herein.

As a non-exhaustive illustration only, a terminal described herein may be a mobile device, such as a cellular phone, a personal digital assistant (PDA), a digital camera, a portable game console, an MP3 player, a portable/personal multimedia player (PMP), a handheld e-book, a portable laptop PC, a global positioning system (GPS) navigation device, a tablet, a sensor, or a stationary device, such as a desktop PC, a high-definition television (HDTV), a DVD player, a Blue-ray player, a set-top box, a home appliance, or any other device known to one of ordinary skill in the art that is capable of wireless communication and/or network communication.

While this disclosure includes specific examples, it will be apparent to one of ordinary skill in the art that various changes in form and details may be made in these examples without departing from the scope of the claims. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner and/or replaced or supplemented by other components. Therefore, the scope of the disclosure is defined not by the detailed description, but by the claims, and all variations within the scope of the claims are to be construed as being included in the disclosure.

## Claims

1. A data processing method performed by a sensor device, the method comprising:
displaying identification information of the sensor device;
encrypting data measured by the sensor device based on the identification information; and
transmitting the encrypted data to a user terminal.

2. The method of claim 1, wherein the displaying comprises:
encoding the identification information to a symbol or a visual code; and
displaying the symbol or the visual code.

3. The method of claim 2, wherein the encoding comprises:
encoding the identification information to at least one of a barcode, a quick response, QR, code, a matrix code, and a color code.

4. The method of one of claims 1 to 3, wherein the encrypting comprises:
setting the identification information as an encryption key; and
encrypting the data based on the encryption key.

5. The method of one of claims 1 to 4, wherein the displaying comprises:
displaying the identification information using non-electronic method.

6. A sensor device adapted for performing data processing, comprising:
means for receiving identification information from a user terminal during a first mode:
means for converting the first mode to a second mode in response to the identification information being received, or a predetermined period of time elapsing;
means for encrypting data measured by the sensor device based on the identification information during a second mode; and
means for transmitting the encrypted data to the user terminal.

7. The sensor device of claim 6, wherein the predetermined period of time begins in response to the sensor device being initialized.

8. The sensor device of claim 6 or 7, wherein the means for encrypting comprises:
means for setting the identification information as an encryption key; and
means for encrypting the data based on the encryption key.

9. A data processing method performed by a user terminal, the method comprising:
reading identification information of a sensor device that is displayed on the sensor device; and
decoding data received from the sensor device based on the identification information.

10. The method of claim 9, wherein the reading comprises:
capturing an image of the identification information displayed on the sensor device; and
extracting the identification information from the image.

11. The method of claim 9 or 10, wherein the reading comprises:
reading the identification information based on a user input.

12. The method of one of claims 9 to 11, wherein the decoding comprises:
decoding the data based on data decoding information corresponding to the identification information.

13. The method of one of claims 9 to 12, wherein the sensor device is configured to encode the identification information as a symbol or a visual code.

14. A user terminal adapted for performing data processing, comprising:
means for transmitting identification information to a sensor device during a first mode;
means for converting the first mode to a second mode in response to the identification information being transmitted, or a predetermined period of time elapsing; and
means for decoding data received from the sensor device based on the identification information during the second mode.

15. The user terminal of claim 14, further comprising:
means for generating the identification information based on a user input.
